(19) Europäisches Patentamt
European Patent Office
Office européen des brevets

(11) EP 2 649 096 B1

(12) **EUROPEAN PATENT SPECIFICATION**

(45) Date of publication and mention of the grant of the patent:
**12.04.2017 Bulletin 2017/15**

(21) Application number: **11846155.7**

(22) Date of filing: **08.12.2011**

(51) Int Cl.:
***C07K 14/805*** *(2006.01)*

(86) International application number:
**PCT/US2011/063921**

(87) International publication number:
**WO 2012/078850 (14.06.2012 Gazette 2012/24)**

# (54) HEMOGLOBIN COMPOSITIONS AND METHODS OF USE

HÄMOGLOBINZUSAMMENSETZUNGEN UND VERFAHREN ZU IHRER VERWENDUNG

COMPOSITIONS D'HÉMOGLOBINE ET PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2010 US 420914 P**

(43) Date of publication of application:
**16.10.2013 Bulletin 2013/42**

(73) Proprietor: **Boston Therapeutics, Inc.**
**Manchester, New Hampshire 03101 (US)**

(72) Inventor: **PLATT, David**
**Newton, Massachusetts 02159 (US)**

(74) Representative: **Lipscombe, Martin John et al**
**Nash Matthews LLP**
**24 Hills Road**
**Cambridge CB2 1JP (GB)**

(56) References cited:
**WO-A1-00/21366**
**US-A- 5 981 710**
**US-A- 5 998 361**
**US-A1- 2002 065 211**
**US-A1- 2005 169 944**

- **EIKE JULIE H ET AL: "Effect of glutaraldehyde concentration on the physical properties of polymerized hemoglobin-based oxygen carriers", BIOTECHNOLOGY PROGRESS, vol. 20, no. 4, July 2004 (2004-07), pages 1225-1232, XP002734641, ISSN: 8756-7938**
- **HARRIS DAVID RAPHAEL ET AL: "Modern Cross-Linking Strategies for Synthesizing Acellular Hemoglobin-Based Oxygen Carriers", BIOTECHNOLOGY PROGRESS, vol. 24, no. 6, November 2008 (2008-11), pages 1215-1225, XP002734642, ISSN: 8756-7938**
- **EIKE JULIE H ET AL: "Oxidized mono-, Di-, Tri-, and polysaccharides as potential hemoglobin cross-linking reagents for the synthesis of high oxygen affinity artificial blood substitutes", BIOTECHNOLOGY PROGRESS, vol. 20, no. 3, May 2004 (2004-05), pages 953-962, XP002734643, ISSN: 8756-7938**
- **MORRA ET AL.: 'Effects on interfacial properties and cell adhesion of surface modification by pectic hairy region.' BIOMACROMOLECULES vol. 5, no. 6, November 2004, pages 2094 - 2104, XP002445360**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 649 096 B1

## Description

### FIELD OF INVENTION

The instant invention relates to oxygen transport agents for the administration to patients for the treatment of hypoxia and related conditions. More specifically, the invention relates to a hybrid hemoglobin molecule that is shielded by the attachment of a carbohydrate molecule to deliver oxygen to cells in a hypoxia condition.

### BACKGROUND OF THE INVENTION

A major component of morbidity and mortality attributable to cardiovascular disease occurs as a consequence of the partial or complete blockage of vessels carrying blood in the coronary and/or peripheral vasculature. When such vessels are partially occluded, lack of blood flow causes ischemia to the muscle tissues supplied by such vessel, consequently inhibiting muscle contraction and proper function. Total occlusion of blood flow causes necrosis of the muscle tissue. Necrosis of muscle tissue causes scar formation, leading to cardiac remodeling and failure.

Blood vessel occlusions are commonly treated by mechanically enhancing blood flow in the affected vessels. Such mechanical enhancements are often provided by employing surgical techniques that attach natural or synthetic conduits proximal and distal to the areas of occlusion, thereby providing bypass grafts, or revascularization by various means to physically enlarge the vascular lumen at the site of occlusion. These revascularization procedures involve such devices as balloons, endovascular knives and endovascular drills. Unfortunately, surgical approaches are accompanied by significant morbidity and even mortality, while the angioplasty-type processes are complicated by recurrent stenoses in many cases.

In some individuals, blood vessel occlusion is partially compensated by natural processes, in which new vessels are formed (termed "angiogenesis") and small vessels are enlarged (termed "arteriogenesis") to replace the function of the impaired vessels. These new vessels may facilitate restoration of blood flow to the deprived tissue, thereby constituting "natural bypasses" around the occluded vessels. However, some individuals are unable to generate sufficient collateral vessels to adequately compensate for the diminished blood flow caused by cardiovascular disease. Accordingly, it would be desirable to provide a composition to compensate for blood loss due to an occlusion in coronary and peripheral arteries in order to treat ischemia. Hemoglobin, as a natural oxygen transporter component of blood, may be in short supply in the areas of occlusion due to these mechanical occlusions causing ischemic conditions. The need for some form of chemical modification of hemoglobin to render it suitable for use as an improved oxygen transporter may compensate for decreased blood supply in ischemic conditions.

### SUMMARY OF INVENTION

According to the disclosure a method and composition for treatment of Hypoxia and related conditions is disclosed. It is contemplated within the scope of the disclosure that ischemia, anemia and trauma can be described as Hypoxia condition. Without being bound to any particular theory it is thought that hypoxia induces transcriptional activation of genes that alter cellular metabolism and promote neo-angiogenesis.

The composition according to the disclosure is a modified hemoglobin oxygen transport agent. According to the disclosure a method and composition are disclosed wherein the composition consists primarily of a carbohydrate shielded hemoglobin (CSH™) to deliver oxygen in hypoxia stage. The composition according to the disclosure is based on oxidation chemistry which is believed to play key roles in ischemia and Anemia and has a general structure as follows:

$$CSH = A[x] - B[y] - C[z]$$

A. Cross linked stabilized Hemoglobin
B. Linkage
C. Natural Pectin derivative

The composition according to the disclosure is a carbohydrate shielded hemoglobin (CSH™) where [x ] units of A are linked to [y] units of B and B is linked to [z] units of C. A is a tetrameric hemoglobin and [x] is 1 and up to 6 tetrameric units. C is a natural derivative of pectin and [z] is 1 and up to 12 units. B is a chemical or physical entities or a combination of these which bridge between A and C, and [y] is 2 and up to 24 defined linkages.

In one aspect of the disclosure the hemoglobin hybrid molecule contains carbohydrates to stabilize the catalytic activity of cross linked tetrameric subunits of hemoglobin protein as a universal carrier for oxygen.

In a further aspect of the disclosure the hemoglobin hybrid molecule, which is injected via IV, circulates in the blood to pick oxygen from the lungs and then targets itself to specific hypoxic organ or injured organ or diseased organ where biomarkers or receptors presented on the membrane surface induce release of the oxygen to the organ. It is contemplated within the scope of the disclosure that other routes of administration are available.

In a further aspect of the disclosure components A and C are bridged through a chemical or/and by physical bridges through multiple interactions of ionic or/and hydrophobic or van der Waals force [or van der Waals interaction]. The bridging forces between the hemoglobin molecules (or between parts of the same molecule) and the natural pectin derivatives due to covalent bonds or/and to the electrostatic interaction or/and van der Waals play fundamental role in the hybrid hemoglobin its superior stability and catalytic activity (oxygen transport).

In another aspect of the disclosure the hemoglobin hybrid molecule penetrates cells and releases oxygen within the cells. Once the cells became oxygenated their hypoxia condition will be reversed.

In yet a further aspect of the disclosure the hemoglobin hybrid molecule has a mechanism of action for direct therapeutic drug intervention for hypoxia. The hybrid hemoglobin molecule according to the disclosure has oxygen affinity that mimics human red blood cells or has greater oxygen transport capacity than human red blood cells and does not cause any adverse effects associate with previous development of modified hemoglobin.

In another aspect of the disclosure the hybrid hemoglobin molecule is compatible with all blood types and can be administer immediately.

In a further aspect of the disclosure the hybrid hemoglobin molecule is a stable composition having a shelf life of about two years.

In another aspect of the disclosure the hybrid hemoglobin molecule can be stored at room temperature with no substantial degradation of the oxygen transport affinity of the composition.

In yet a further aspect of the disclosure the modified hemoglobin molecule can be adapted for therapeutic efficacy for treatments of degenerative diseases associate with hypoxia.

In another aspect of the invention it is thought that the growth and metastasis of solid tumors is critically dependent on tumor angiogenesis. A major stimulus for a tumor's recruitment of additional blood vessels is cellular hypoxia. Hypoxia induces transcriptional activation of genes that alter cellular metabolism and promote neo-angiogenesis. The tumors in most end stage cancer patients are in a hypoxia state and are dividing slowly and not responding to most anti cancer drugs. It is therefore an aspect of the invention to provide the composition according to the disclosure to prevent neo-angiogenesis and restore normal cancer cell division that will allow anti cancer drug to work more effectively.

In yet a further aspect of the disclosure the hybrid hemoglobin molecule targets itself to specific receptors on the membrane surface of cancers cells thereby penetrating the cells and releases oxygen within the cancer cells. Once the cancer cells become oxygenated and their hypoxia condition is reversed anticancer drugs will have a greater rate of efficiency.

BRIEF DESCRIPTION OF THE DRAWINGS

The embodiments described in the present disclosure are illustrated in the figures of the accompanying drawings which are meant to be exemplary and not limiting, in which like references are intended to refer to like or corresponding parts, and in which:

Figure 1 illustrates the structure of hemoglobin including the positions of the alpha and beta subunits.

DETAILED DESCRIPTION

The hemoglobin for use in the composition of the present disclosure is preferably human hemoglobin, derived from red blood cells. However, the invention is applicable also to other types of hemoglobin to form the basis of an oxygen transport agent, such as animal hemoglobin especially bovine hemoglobin, and porcine hemoglobin and the like, and hemoglobin derived from cell culture. The hybrid hemoglobin molecule according to the disclosure has a general structure as follows:

$$CSH = A[x] - B[y] - C[z]$$

A: Cross linked stabilized Hemoglobin
B: Linkage
C: Natural Pectin derivative

The composition according to the disclosure is a carbohydrate shielded hemoglobin (CSH™) where [X ] units of A are linked to [y] units of B and B is linked to [z] units of C. A is a tetrameric hemoglobin and [x] is 1 and up to 6 tetrameric units. C is a natural derivative of pectin and [z] is 1 and up to 12 units. B is a chemical or physical entity or a combination of these which bridge between A and C, and [y] is 2 and up to 24 defined linkages.

**Component A:**

A is a cross linked stabilized hemoglobin Structure

**Hemoglobin Structure**

The oxygen-carrying protein hemoglobin was discovered by Hünefeld in 1840. In 1851, Otto Funke published a series of articles in which he described growing hemoglobin crystals by successively diluting red blood cells with a solvent such as pure water, alcohol or ether, followed by slow evaporation of the solvent from the resulting protein solution.

Hemoglobin's reversible oxygenation was described a few years later by Felix Hoppe-Seyler. The role of hemoglobin in the blood was elucidated by physiologist Claude Bernard. The name *hemoglobin* is the portmanteau of *heme* and globin, reflecting the fact that each subunit of hemoglobin is a globular protein with an embedded heme (or haem) group. Each heme group contains one iron atom that can bind one oxygen molecule through ion-induced dipole forces. The most common type of hemoglobin in mammals contains four such subunits.

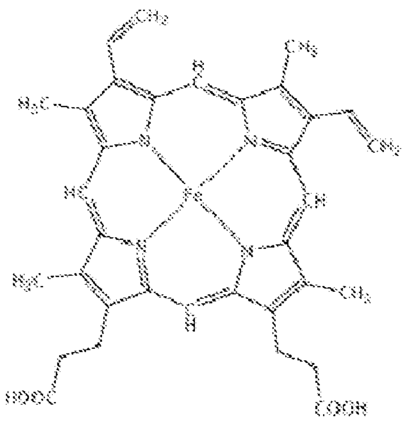

Heme group

In most humans, the hemoglobin molecule is an assembly of four globular protein subunits. Each subunit is composed of a protein chain tightly associated with a non-protein heme group. Each protein chain arranges into a set of alpha-helix structural segments connected together in a globin fold arrangement, so called because this arrangement is the same folding motif used in other heme/globin proteins such as myoglobin. This folding pattern contains a pocket which strongly binds the heme group.

A heme group consists of an iron (Fe) ion (charged atom) held in a heterocyclic ring, known as a porphyrin. The iron ion, which is the site of oxygen binding, coordinates with the four nitrogens in the center of the ring, which all lie in one plane. The iron is also bound strongly to the globular protein via the imidazole ring of the F8 histidine residue below the porphyrin ring. A sixth position can reversibly bind oxygen by a coordinate covalent bond, completing the octahedral group of six ligands. Oxygen binds in an "end-on bent" geometry where one oxygen atom binds Fe and the other protrudes at an angle. When oxygen is not bound, a very weakly bonded water molecule fills the site, forming a distorted octahedron.

The iron ion may either be in the $Fe^{2+}$ or $Fe^{3+}$ state, but ferrihemoglobin (methemoglobin) ($Fe^{3+}$) cannot bind oxygen. In binding, oxygen temporarily oxidizes ($Fe^{2+}$) to ($Fe^{3+}$), so iron must exist in the +2 oxidation state in order to bind oxygen. The enzyme methemoglobin reductase reactivates hemoglobin found in the inactive ($Fe^{3+}$) state by reducing the iron center.

In adult humans, the most common hemoglobin type is a tetramer (which contains 4 subunit proteins) called **hemoglobin A,** consisting of two $\alpha$ and two $\beta$ subunits non-covalently bound, each made of 141 and 146 amino acid residues, respectively. This is denoted as $\alpha_2\beta_2$. The subunits are structurally similar and about the same size. Each subunit has a molecular weight of about 17,000 daltons, for a total molecular weight of the tetramer of about 68,000 daltons (64,458 g/mol). 1 g/dL = 0.6206 mmol/L. Hemoglobin A is the most intensively studied of the hemoglobin molecules.

The four polypeptide chains are bound to each other by salt bridges, hydrogen bonds, and hydrophobic interactions. There are two kinds of contacts between the $\alpha$ and $\beta$ chains: $\alpha_1\beta_1$ and $\alpha_1\beta_2$.

*Oxyhemoglobin* is formed during respiration when oxygen binds to the heme component of the protein hemoglobin in red blood cells. This process occurs in the pulmonary capillaries adjacent to the alveoli of the lungs. The oxygen then travels through the blood stream to be dropped off at cells where it is utilized in aerobic glycolysis and in the production of ATP by the process of oxidative phosphorylation. It does not, however, help to counteract a decrease in blood pH. Ventilation, or breathing, may reverse this condition by removal of carbon dioxide, thus causing a shift up in pH.

*Deoxyhemoglobin* is the form of hemoglobin without the bound oxygen. The absorption spectra of oxyhemoglobin and deoxyhemoglobin differ. The oxyhemoglobin has significantly lower absorption of the 660 nm wavelength than deoxyhemoglobin, while at 940 nm its absorption is slightly higher. This accounts for hemoglobin's red color and deoxyhemoglobin's blue color. This difference is used for measurement of the amount of oxygen in patient's blood by an instrument called pulse oximeter.

Chemically connected protein arrays have significant diverse applications including the production of red cell substitutes, bioconjugate drug delivery, and protein therapies. (See Efficient generation of dendritic arrays of cross-linked hemoglobin: symmetry and redundancy, Dongxin Hu and Ronald Kluger).

In order to make materials of defined structure, there is a need for efficient and accessible reagents. While chemical cross-linking with a multi-subunit protein can be achieved in high yield, connecting proteins to one another in a dendritic assembly along with concurrent cross-linking has met with limited success. This has now been overcome through the design and implementation of a readily prepared reagent with added reaction sites that compensate for competing hydrolysis. *N,N',N"*-Tris[bis(sodium methyl phosphate)isophthalyl]-1,3,5-benzenetricarboxamide (**1**), a hexakis(methyl phosphate) isophthalyl trimesoyl tris-amide, was designed and synthesized in high yield in three stages from a reactive trimesoyl core.

This material has three pairs of coplanar cross-linking reaction sites in a symmetrical array. The presence of three sets of sites greatly increases the probability that at least two sets will produce cross-links within hemoglobin tetramers (in competition with hydrolysis) and thereby connect two cross-linked tetramers at the same time. Reaction of **1** with deoxyhemoglobin at pH 8.5 gives a material that contains two cross-linked hemoglobin tetramers connected to one another and to a constituent αßdimer. Products were characterized by SDS-PAGE, MS, enzyme digestion and HPLC. The isolated dendritic-hemoglobin with 2.5 tetrameric components has the same oxygen affinity as native hemoglobin ($P_{50}$ = 5.0 torr) and retains cooperativity ($n_{50}$ = 2.0). Analysis of circular dichroism spectra indicates that the assembly retains proper folding of the globin chains while the hemes are in an altered environment.

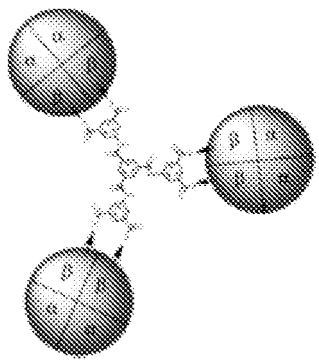

SH is made of chemically stabilized, cross-linked bovine (cow) hemoglobin situated in a salt solution. It is contemplated within the scope of the disclosure that other sources of hemoglobin can be used including human hemoglobin, cell culture hemoglobin and other animal derived hemoglobin. Hemoglobin by itself is toxic to the kidneys because it contains stroma lipids, which are contaminated with endotoxins. If the stroma lipids are removed, however, then the Hemoglobin has too high an affinity for oxygen, which means less oxygen off-loading to the tissues. In order to assure that the Hemoglobin is not toxic, but still therapeutically useful, it must be stabilized. Stabilization can be achieved by cross-linking the hemoglobin. This is done by cross-linking the two alpha, and the two beta subunits. This then stabilizes the alpha-beta dimers, which in turn makes the hemoglobin molecule more stable, and also reduces its affinity for oxygen, making it easier to deliver oxygen to the tissues. The structure of hemoglobin is depicted in Fig. 1, including the positions of the alpha and beta subunits.

## Safety Measures

Since stabilized hemoglobin used according to the disclosure is derived from cows, the instant hybrid hemoglobin

molecule goes through extensive lengths to make sure that it is: free of pathogens, infectious agents (BSE), and chemically pure, including: using only very tightly monitored herds, where the country of origin, food supply and health are closely supervised; the use of external experts who closely regulate the strict manufacturing process of the hybrid hemoglobin molecule to determine the capability to remove potential pathogens; and strict observance of the global industry and regulatory standards.

**Advantages of hybrid hemoglobin molecule vs. RBC's**

The advantages of the hybrid hemoglobin molecule according to the disclosure over that of traditional source red blood cells (RBC) as an oxygen transport agent are shown in Table 1 below:

Table 1

| CHARACTERISTICS | HYBRID HEMOGLOBIN MOLECULES | RED BLOOD CELLS |
|---|---|---|
| STORAGE | *Room temperature (2° to 30° C)* | Refrigerated |
| SHELF LIFE | *36 months* | 42 days |
| PREPARATION | *Ready to use* | Testing, typing and crossmatching |
| COMPATIBILITY | *Universal* | Type specific |
| EFFECTIVENESS | *Immediate oxygen delivery* | Dependent on length of storage |
| PURITY | *Processed to remove infectious agents* | Tested and screened for infectious agents |
| RAW MATERIALS | *Bovine hemoglobin – abundant, controlled source* | Limited availability, not controlled |

The hybrid hemoglobin molecule according to the disclosure is smaller in size (up to about 1,000 times smaller than a typical red blood cell) and has less viscosity than human red blood cells (which contain hemoglobin). This means that it can carry more oxygen at a lower blood pressure than red blood cells. Also, because of its smaller size, it can carry oxygen through partially obstructed or restricted blood vessels, where RBC's cannot reach.

Inadequate tissue oxygenation resulting from occluded arteries can result in heart attack, angina, or transient ischemic attack, which is a precursor to stroke. Traditionally, these conditions have been treated by blood transfusions, but RBC's are often too big to pass through the occlusion, which is why the hybrid hemoglobin molecule according to the disclosure can potentially be therapeutically beneficial.

A vertebrate, having a localized, regional or systemic reduction in RBC flow, can have oxygen transport systems which are otherwise normal, or can have additional abnormalities which can deleteriously affect oxygen transport and transfer in a portion of the body, or throughout the body as a whole.

In addition, in this method the vertebrate has a normovolemic blood volume prior to administration of the hemoglobin. A normovolemic blood volume is defined as a volume of blood within the circulatory system of the vertebrate which will not result in hypovolemic shock, such as can result from a major hemorrhage or a large loss of fluid secondary to vomiting, diarrhea, bums or dehydration. Typically, a normovolemic blood volume includes at least about 90% of the normal volume of blood for that vertebrate. In some cases a normovolemic volume can contain as little as about 80% of the normal blood volume without resulting in hypovolemic shock.

Furthermore, the blood constituting the normovolemic blood volume, contains at least about a normal concentration of RBCs. For example, the blood in a normovolemic blood volume of a human typically has a major vessel hematocrit of at least about 30%.

In this method, a vertebrate also has a normal, or higher than normal, systemic vascular resistance in the circulatory system, prior to administering the hemoglobin. A normal systemic vascular resistance is a vascular resistance which would not result in distributive shock, such as septic shock, in the vertebrate.

Reduced red blood cell flow includes any reduction in RBC flow, localized, regionalized and/or systemic, below normal RBC flow levels, including a "no RBC flow" condition. Localized RBC flow consists of RBC flow through one or more capillaries within a capillary bed, wherein said capillaries would normally provide RBC flow to oxygenate a localized tissue area. Regionalized RBC flow provides RBC flow to oxygenate a larger tissue area, such as a limb or organ. Systemic RBC flow is flow through the major circulatory systems of the body, thus providing RBCs to oxygenate the body as a whole.

In one illustrative embodiment of the method of disclosure, hybrid hemoglobin molecule (HHM) is administered to a vertebrate who has, or will have, a partial obstruction of the circulatory system, such as a stenosis or vascular blockage, in an amount that reduces or precludes RBC flow past the partial obstruction, but by which at least some plasma can flow. Administering HHM increases tissue oxygenation in tissue distal to a localized or regionalized partial obstruction, and/or to increases tissue oxygenation throughout the body to treat a systemic partial obstruction.

RBCs are significantly larger than hybrid hemoglobin molecule according to the disclosure, typically being 7-10

microns in diameter, therefore requiring significantly larger vascular openings, than does HHM, to flow past a partial obstruction.

Partial obstructions can occur at all tissue locations and in all blood vessels, such as arteries, veins and capillaries. In addition, valves within the circulatory system, such as aortic, mitral and tricuspid valves, can also be partially obstructed. Further, chamber or sections of the heart can be partially obstructed, such as ventricular outflows and the ventricular opening to the pulmonary artery.

A partial obstruction of the circulatory system can be temporary, permanent or recurrent. A circulatory system partial obstruction can be caused by various means, such as vessel wall defects, disease, injury, aggregation of blood components, neoplasms, space-occupying lesions, infections, foreign bodies, compression, drugs, mechanical devices, vasoconstriction and vasospasms.

A stenosis of the circulatory system, as defined herein, is a narrowing of any canal, or lumen, in the circulatory system. Typically, a stenosis can result from disease, such as atherosclerosis; a vessel wall abnormality, such as a suture line from an arterial graft, a junction point of attachment for a graft or stent, a kink or deformity in a vessel, graft or stent, healed or scarred tissue from an injury or invasive procedure (e.g., catheterization, angioplasty, vascular stenting, vascular grafting with prosthesis, allogenic tissue and/or autologous tissue); a vascular prosthesis such as an artificial valve or vessel; compression, such as by a neoplastic mass, hematoma or mechanical means (e.g., clamp, tourniquet or cuff device); chemical poisoning or drug side effects; vasoconstriction; and vasospasms.

**Component B:**

B is the 'bridge' 'linker' between A and C.

In one illustrative embodiment components A and C are liked by the several common linking technologies that include but are not limited to Thermo Scientific Pierce Sulfo-NHS (*N*-hydroxysulfosuccinimide) and its uncharged analog NHS (*N*-hydroxysuccinimide) are chemical modification reagents for converting carboxyl groups to amine-reactive Sulfo-NHS esters for application in a variety of bioconjugation and cross-linking methods.

In one illustrative embodiment components A and C are liked by physical bridges in addition through multiple interactions of ionic or/and hydrophobic or van der Waals force [(or van der Waals interaction). The attractive or repulsive force between molecules (or between parts of the same molecule) other than those due to covalent bonds or to the electrostatic interaction of ions with one another or with neutral molecules. The term includes: force between permanent dipole and a corresponding induced dipole instantaneous induced dipole-induced dipole (London dispersion force). These forces may play a fundamental role in hybrid hemoglobin biological catalytic activity and polymer stability.

Chemical bonds are easily synthesized by mixing the Sulfo-NHS with a carboxyl-containing molecule and a dehydrating agent such as the carbodiimide EDC (EDAC). The method is the basis for generating many types of protein labeling reagents, including amine-reactive fluorescent dyes, biotin affinity tags and pegylation compounds.

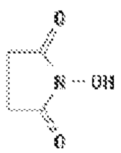

NHS
M.W. 115.09

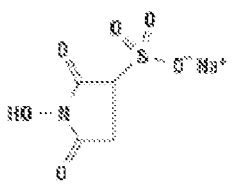

Sulfo-NHS
M.W. 217.13

Structures of NHS and Sulfo-NHS chemical modification reagents.

In a further illustrative embodiment a noncleavable, membrane-impermeable, water-soluble crosslinking agent is used as a linking agent. This linking agent is as follows:

Bis(sulfosuccinimidyl)suberate ($BS^3$) is a homobifunctional, water-soluble, non-cleavable and membrane impermeable crosslinker. It contains an amine-reactive *N*-hydroxysulfosuccinimide (NHS) ester at each end of an 8-carbon spacer arm. NHS esters react with primary amines at pH 7-9 to form stable amide bonds, along with release of the N-hydroxysulfosuccinimide leaving group. Proteins, including antibodies, generally have several primary amines in the side chain of lysine (K) residues and the N-terminus of each polypeptide that are available as targets for NHS-ester crosslinking reagents.

Because it contains the hydrophilic sulfonyl moiety, $BS^3$ crosslinker is soluble up tao ~10 mM in water and many commonly used buffers, thus avoiding the use of organic solvents which may perturb protein structure. DSS, the non-water soluble analog of $BS^3$ is also available for applications that require a less hydrophilic crosslinker (e.g., to effect

intracellular crosslinking). DSS and $BS^3$ have essentially identical crosslinking activity toward primary amines.

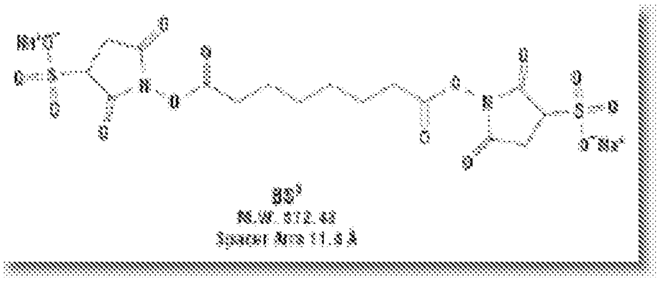

**Component C: Natural Pectin derivative**

The characteristic structure of natural pectin is a linear chain of α-(1-4)-linked D-galacturonic acid that forms the pectin-backbone, a homogalacturonan. Pectin is a polysaccharide that acts as a cementing material in the cell walls of all plant tissues. The white portion of the rind of lemons and oranges contains approximately 30% pectin. Natural pectin is the methylated ester of polygalacturonic acid, which consists of chains of 300 to 1000 galacturonic acid units joined with 1α→4 linkages. The Degree of Esterification (DE) affects the gelling properties of pectin. The structure shown here has three methyl ester forms (**-$COOCH_3$**) for every two carboxyl groups (**-COOH**), hence it is has a 60% degree of esterification, normally called a DE-60 pectin.

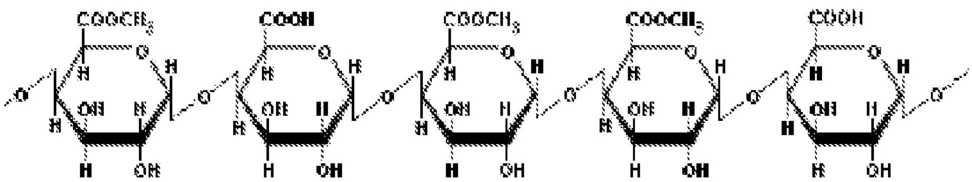

Pectin is a polymer of α-Galacturonic acid with a variable number of methyl ester groups.

Into this backbone, there are regions where galacturonic acid is replaced by (1-2)-linked L-rhamnose. From the rhamnose residues, side-chains of various neutral sugars branch off. This type of pectin is called rhamnogalacturonan I. Up to every 25th galacturonic acid in the main chain is replaced with rhamnose. Some stretches consist of alternating galacturonic acid and rhamnose - "hairy regions", others with lower density of rhamnose - "smooth regions". The neutral sugars are mainly D-galactose, L-arabinose and D-xylose, the types and proportions of neutral sugars varying with the origin of pectin.

A third structural type of pectin is rhamnogalacturonan II, which is a less frequent complex, highly branched polysaccharide.

Isolated natural pectin has a molecular weight of typically up to 400,000 g/mol, varying with origin and extraction conditions.

In nature, around 80% of carboxyl groups of galacturonic acid are esterified with methanol. This proportion is decreased more or less during pectin extraction. The ratio of esterified to non-esterified galacturonic acid determines the behavior of pectin in food applications. This is why pectins are classified as high- vs. low-ester pectins - or in short HM vs. LM-pectins, with more or less than half of all the galacturonic acid esterified.

The non-esterified galacturonic acid units can be either free acids (carboxyl groups) or salts with sodium, potassium or calcium. The salts of partially esterified pectins are called pectinates, if the degree of esterification is below 5% the salts are called pectates, the insoluble acid form, pectic acid.

Amidated pectin is a modified form of pectin. Here, some of the galacturonic acid is converted with ammonia to carboxylic acid amide. These pectins are more tolerant of varying calcium concentrations that occur in use.

High-ester pectins set at higher temperatures than low-ester pectins. However, gelling reactions with calcium increase as the degree of esterification falls. Similarly, lower pH-values or higher soluble solids (normally sugars) increase gelling speed.

## Claims

1. A method for producing a hybrid hemoglobin molecule, useful for forming a stable blood-substitute, from hemoglobin contained in a hemoglobin solution, comprising the steps of:

a) deoxygenating said hemoglobin solution;
b) mixing said deoxygenated hemoglobin solution with a sulfhydryl compound;
c) mixing said solution of sulfhydryl compound and hemoglobin with a cross-linking agent to form a polymerization reaction mixture;
d) polymerizing the polymerization reaction mixture, thereby forming a polymerized hemoglobin solution;
e) reacting said polymerized hemoglobin solution with linking agents thereby converting carboxyl groups to amine-reactive Sulfo-NHS esters forming a precursor reactive molecule; and
f) reacting said precursor reactive molecule with a polysaccharide, wherein the polysaccharide is a natural pectin derivative

**2.** The method according to claim 1 wherein said hemoglobin solution comprises mammalian hemoglobin.

**3.** The method according to claim 1 wherein said mammalian hemoglobin solution is formed from hemoglobin selected from a group consisting of human hemoglobin, bovine hemoglobin, ovine hemoglobin, porcine hemoglobin.

**4.** The method according to claim 1 wherein the deoxygenated hemoglobin solution has an oxyhemoglobin content of less than about ten percent.

**5.** The method according to claim 1 wherein the polysaccharide is a substantially methoxylated rhamongalacturonic acid.

**6.** The method according to claim 1 wherein said sulfhydryl compound is selected from the group consisting of N-acetyl-L-cysteine, D,L-cysteine, glutathione, gamma -glutamyl -cysteine, 2,3-dimercapto-1-propanol, thioglycolate, and 1 ,4-butanedithiol, or a general formula thiol-[CHO]n-thiol, to form a solution of the sulfhydryl compound and said hemoglobin.

**7.** The method of claim 1 wherein said hybrid hemoglobin molecule is a carbohydrate shielded stabilized hemoglobin.

**8.** Use of a hybrid haemoglobin molecule, made by the method of claim 1, for treating a hypoxic organ, which has oxygen heterogeneity, due to uncontrolled proliferation.

**9.** Use of a hybrid haemoglobin molecule, made by the method of claim 1, for increasing tissue oxygenation and, consequently, increasing organ function of a vertebrate, while the organ has reduced oxygen delivery due to at least one partial obstruction of a blood vessel within the circulatory system of the vertebrate, and wherein the vertebrate has a normovolemic blood volume and at least a normal systemic vascular resistance.

**10.** The use of claim 8 wherein said hybrid hemoglobin molecule is a carbohydrate shielded stabilized hemoglobin.

**11.** The use of claim 8 wherein the organ is a traumatic organ selected from the group consisting of muscle, heart, brain, lung and skin

**12.** The use of claim 11 wherein the heart has a partial stenosis selected from the group consisting of a blood vessel stenosis, a valve stenosis, a stenosis of an opening in the heart and a stenosis of a chamber of the heart.

**13.** Use of a hybrid haemoglobin molecule, made by the method of claim 1, for increasing tissue oxygenation and, consequently, increasing organ function of a vertebrate, while the organ has reduced oxygen delivery due to at least one partial obstruction of a blood vessel within the circulatory system of the vertebrate, and wherein the vertebrate has a major vessel hematocrit of at least about 30% and at least a normal systemic vascular resistance.

**Patentansprüche**

**1.** Verfahren zur Herstellung eines Hybrid-Hämoglobinmoleküls, das zur Bildung eines stabilen Blutersatzes aus Hämoglobin brauchbar ist, das in einer Hämoglobinlösung enthalten ist, umfassend die Schritte:

a) Deoxygenieren der Hämoglobinlösung;
b) Mischen der deoxygenierten Hämoglobinlösung mit einer Sulfhydrylverbindung;
c) Mischen der Lösung der Sulfhydrylverbindung und des Hämoglobins mit einem Vernetzungsmittel, um eine

Polymerisationsreaktionsmischung zu bilden;
d) Polymerisieren der Polymerisationsreaktionsmischung, wodurch eine polymerisierte Hämoglobinlösung gebildet wird;
e) Umsetzen der polymerisierten Hämoglobinlösung mit Verknüpfungsmitteln, wodurch Carboxylgruppen in aminreaktive Sulfo-NHS-Ester umgewandelt werden, die ein reaktives Vorläufermolekül bilden; und
f) Umsetzen des reaktiven Vorläufermoleküls mit einem Polysaccharid, wobei das Polysaccharid ein natürliches Pektinderivat ist.

**2.** Verfahren nach Anspruch 1, wobei die Hämoglobinlösung Säuger-Hämoglobin umfasst.

**3.** Verfahren nach Anspruch 1, wobei die Säuger-Hämoglobinlösung aus Hämoglobin gebildet ist, das ausgewählt ist aus einer Gruppe bestehend aus Hämoglobin des Menschen, Hämoglobin des Rinds, Hämoglobin des Schafs und Hämoglobin des Schweins.

**4.** Verfahren nach Anspruch 1, wobei die deoxygenierte Hämoglobinlösung einen Oxyhämoglobingehalt von weniger als etwa zehn Prozent aufweist.

**5.** Verfahren nach Anspruch 1, wobei das Polysaccharid eine im Wesentlichen methoxylierte Rhamnogalacturonsäure ist.

**6.** Verfahren nach Anspruch 1, wobei die Sulfhydrylverbindung ausgewählt ist aus der Gruppe bestehend aus N-Acetyl-L-cystein, D,L-Cystein, Glutathion, gamma-Glutamylcystein, 2,3-Dimercapto-1-propanol, Thioglycolat und 1,4-Butandithiol oder einer allgemeinen Formel Thiol-$[CHO]_n$-thiol, um eine Lösung der Sulfhydrylverbindung und des Hämoglobins zu bilden.

**7.** Verfahren nach Anspruch 1, wobei das Hybrid-Hämoglobinmolekül ein kohlenhydratabgeschirmtes stabilisiertes Hämoglobin ist.

**8.** Verwendung eines Hybrid-Hämoglobinmoleküls, das nach dem Verfahren gemäß Anspruch 1 hergestellt ist, zur Behandlung eines hypoxischen Organs, das infolge von unkontrollierter Proliferation Sauerstoffheterogenität aufweist.

**9.** Verwendung eines Hybrid-Hämoglobinmoleküls, das nach dem Verfahren gemäß Anspruch 1 hergestellt ist, zur Erhöhung der Oxygenierung von Gewebe und somit zur Erhöhung der Organfunktion eines Wirbeltiers, während das Organ infolge von mindestens einer Teilobstruktion eines Blutgefäßes im Kreislaufsystem des Wirbeltiers reduzierte Sauerstoffzufuhr aufweist, und wobei das Wirbeltier ein normovolämisches Blutvolumen und mindestens einen normalen systemischen Gefäßwiderstand aufweist.

**10.** Verwendung nach Anspruch 8, wobei das Hybrid-Hämoglobinmolekül ein kohlenhydratabgeschirmtes stabilisiertes Hämoglobin ist.

**11.** Verwendung nach Anspruch 8, wobei das Organ ein traumatisiertes Organ ausgewählt aus der Gruppe bestehend aus Muskel, Herz, Hirn, Lunge und Haut ist.

**12.** Verwendung nach Anspruch 11, wobei das Herz eine Teilstenose ausgewählt aus der Gruppe bestehend aus einer Blutgefäßstenose, einer Klappenstenose, einer Stenose einer Öffnung in dem Herzen und einer Stenose einer Kammer des Herzens aufweist.

**13.** Verwendung eines Hybrid-Hämoglobinmoleküls, das nach dem Verfahren gemäß Anspruch 1 hergestellt ist, zur Erhöhung der Oxygenierung von Gewebe und somit zur Erhöhung der Organfunktion eines Wirbeltiers, während das Organ infolge von mindestens einer Teilobstruktion eines Blutgefäßes im Kreislaufsystem des Wirbeltiers reduzierte Sauerstoffzufuhr aufweist, und wobei das Wirbeltier einen Hämatokrit der Hauptgefäße von mindestens etwa 30 % und mindestens einen normalen systemischen Gefäßwiderstand aufweist.

## Revendications

**1.** Procédé de production d’une molécule d’hémoglobine hybride, utile pour former un substitut de sang stable, à partir

d'hémoglobine contenue dans une solution d'hémoglobine, comprenant les étapes de :

a) désoxygénation de ladite solution d'hémoglobine ;
b) mélange de ladite solution d'hémoglobine désoxygénée avec un composé sulfhydryle ;
c) mélange de ladite solution de composé sulfhydryle et d'hémoglobine avec un agent de réticulation pour former un mélange de réaction de polymérisation ;
d) polymérisation du mélange de réaction de polymérisation, de manière à former une solution d'hémoglobine polymérisée ;
e) réaction de ladite solution d'hémoglobine polymérisée avec des agents de liaison de manière à convertir les groupes carboxyle en esters Sulfo-NHS réactifs avec les amines de manière à former une molécule réactive de précurseur ; et
f) réaction de ladite molécule réactive de précurseur avec un polysaccharide, le polysaccharide étant un dérivé naturel de pectine.

**2.** Procédé selon la revendication 1 dans lequel ladite solution d'hémoglobine comprend de l'hémoglobine de mammifère.

**3.** Procédé selon la revendication 1 dans lequel ladite solution d'hémoglobine de mammifère est formée à partir d'hémoglobine choisie dans un groupe constitué de l'hémoglobine humaine, l'hémoglobine bovine, l'hémoglobine ovine, l'hémoglobine porcine.

**4.** Procédé selon la revendication 1 dans lequel la solution d'hémoglobine désoxygénée possède une teneur en oxyhémoglobine inférieure à environ dix pour cent.

**5.** Procédé selon la revendication 1 dans lequel le polysaccharide est un acide rhamnogalacturonique sensiblement méthoxylé.

**6.** Procédé selon la revendication 1 dans lequel ledit composé sulfhydryle est choisi dans le groupe constitué de la N-acétyl-L-cystéine, la D,L-cystéine, le glutathion, la gamma-glutamyl-cystéine, le 2,3-dimercapto-1-propanol, le thioglycolate, et le 1,4-butanedithiol, ou une formule générale thiol-[CHO]n-thiol, pour former une solution du composé sulfhydryle et de ladite hémoglobine.

**7.** Procédé de la revendication 1 dans lequel ladite molécule d'hémoglobine hybride est une hémoglobine stabilisée protégée par glucide.

**8.** Utilisation d'une molécule d'hémoglobine hybride, préparé par le procédé de la revendication 1, pour traiter un organe hypoxique, qui présente une hétérogénéité d'oxygène, en raison d'une prolifération incontrôlée.

**9.** Utilisation d'une molécule d'hémoglobine hybride, préparée par le procédé de la revendication 1, pour augmenter l'oxygénation tissulaire et, par conséquent, augmenter la fonction d'organe d'un vertébré, l'organe ayant un approvisionnement réduit en oxygène en raison d'au moins une obstruction partielle d'un vaisseau sanguin dans le système circulatoire du vertébré, et le vertébré ayant un volume sanguin normovolémique et au moins une résistance vasculaire systémique normale.

**10.** Utilisation de la revendication 8 dans laquelle ladite molécule d'hémoglobine hybride est une hémoglobine stabilisée protégée par glucide.

**11.** Utilisation de la revendication 8 dans laquelle l'organe est un organe traumatique choisi dans le groupe constitué des muscles, du coeur, du cerveau, du poumon et de la peau.

**12.** Utilisation de la revendication 11 dans laquelle le coeur présente une sténose partielle choisie dans le groupe constitué d'une sténose de vaisseau sanguin, une sténose valvulaire, une sténose d'une ouverture dans le coeur et une sténose d'une chambre du coeur.

**13.** Utilisation d'une molécule d'hémoglobine hybride, préparée par le procédé de la revendication, pour augmenter l'oxygénation tissulaire et, par conséquent, augmenter la fonction d'organe d'un vertébré, l'organe ayant une un approvisionnement réduit en oxygène en raison d'au moins une obstruction partielle d'un vaisseau sanguin dans le système circulatoire du vertébré, et le vertébré ayant un hématocrite vasculaire majeur d'au moins environ 30 %

et au moins une résistance vasculaire systémique normale.

A

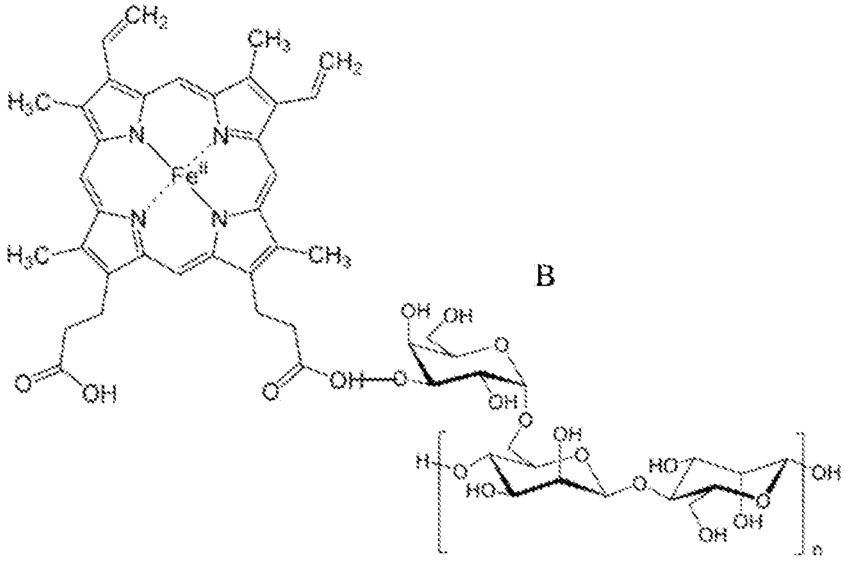

B

C

Figure 1